(19) Europäisches Patentamt
European Patent Office
Office européen des brevets

(11) Veröffentlichungsnummer: **0 356 797**
**A2**

# (12) EUROPÄISCHE PATENTANMELDUNG

(21) Anmeldenummer: **89114980.9**

(22) Anmeldetag: **12.08.89**

(51) Int. Cl.[5]: **C07D 213/64 , C07D 213/73 , //C07C43/23,A01N43/40**

(30) Priorität: **25.08.88 DE 3828820**

(43) Veröffentlichungstag der Anmeldung: **07.03.90 Patentblatt 90/10**

(84) Benannte Vertragsstaaten: **AT BE CH DE ES FR GB IT LI NL**

(71) Anmelder: **BAYER AG**

**D-5090 Leverkusen 1 Bayerwerk(DE)**

(72) Erfinder: **Alig, Bernd, Dr.**
**Gartenstrasse 2**
**D-5330 Königswinter 21(DE)**
Erfinder: **Stendel, Wilhelm, Dr.**
**In den Birken 55**
**D-5600 Wuppertal 1(DE)**
Erfinder: **Londershausen, Michael, Dr.**
**Galileistrasse 11**
**D-4006 Erkrath(DE)**

## (54) Substituierte Pyridine, Verfahren zu ihrer Herstellung und ihre Verwendung als Insektizide.

(57) Die vorliegende Erfindung betrifft substituierte Pyridine der Formel

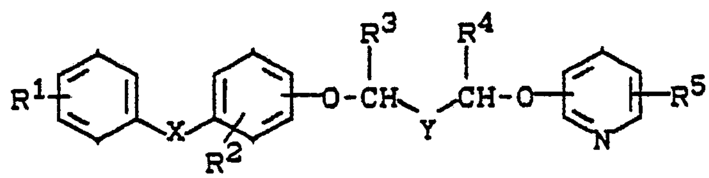

(I)

in welcher
Z für O, S, NH, $-CH_2-$, $-CHCH_3-$, $-C(CH_3)_2-$ steht,
Y für geradkettiges oder verzweigtes Alkylen, das gegebenenfalls durch Sauerstoff unterbrochen ist, steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam für eine direkte Bindung stehen können,
$R^5$ für Wasserstoff, Halogen, Alkyl, Halogenalkyl, Alkoxy, Amino, Alkyl, Alkylamino, Dialkylamino Acylamino steht,
sowie deren Salze mit Säuren, Verfahren zu ihrer Herstellung sowie ihre Verwendung zur Bekämpfung von Insekten, insbesondere Flöhen.

EP 0 356 797 A2

## Substituierte Pyridine, Verfahren zu ihrer Herstellung und ihre Verwendung als Insektizide

Die vorliegende Erfindung betrifft substituierte Pyridine, Verfahren zu ihrer Herstellung, Zwischenprodukte dafür sowie ihre Verwendung als Insektizide.

1. Es wurden die neuen substituierten Pyridine der Formel I gefunden

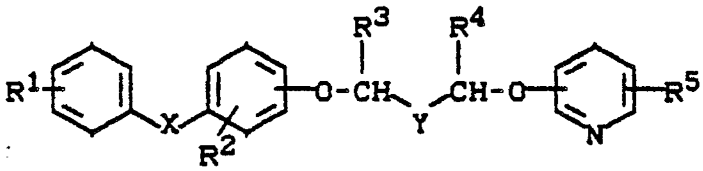

(I)

in welcher
X für O, S, NH, $-CH_2-$, $-CHCH_3-$, $-C(CH_3)_2-$steht,
Y für geradkettiges oder verzweigtes Alkylen, das gegebenenfalls durch Sauerstoff unterbrochen ist, steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam für eine direkte Bindung stehen können,
$R^5$ für Wasserstoff, Halogen, Alkyl, Halogenalkyl, Alkoxy, Amino, Alkyl, Alkylamino, Dialkylamino, Acylamino steht,
sowie deren Salze mit Säuren.

2. Es wurden Verfahren zur Herstellung der substituierten Pyridine der Formel I gefunden, die dadurch gekennzeichnet sind, daß man

a) Verbindungen der Formel II

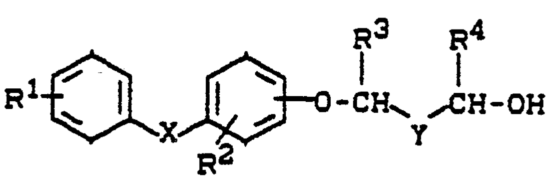

II

in welcher
$R^1$, $R^2$, $R^3$, $R^4$, X, Y die oben angegebene Bedeutung haben,
mit Halogenpyridinen der Formel III

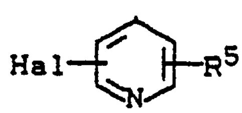

III

in welcher
Hal für Halogen steht und
$R^5$ die oben angegebene Bedeutung hat,
umsetzt, oder

b) Verbindungen der Formel IV

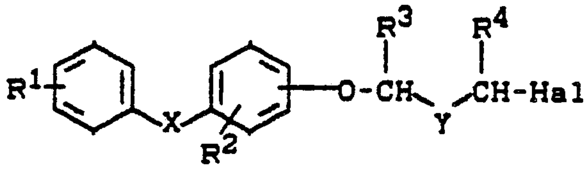

IV

in welcher
$R^1$, $R^2$, $R^3$, $R^4$, X, Y die oben angegebene Bedeutung haben, und
Hal für Halogen steht,
mit Hydroxypyridinen der Formel V

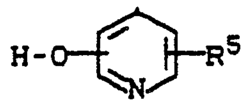

V

in welcher
$R^5$ die oben angegebene Bedeutung hat,
umsetzt.

3. Die Verbindungen der Formel II

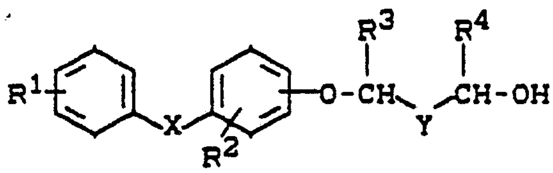

II

in welcher
$R^1$, $R^2$, $R^3$, $R^4$, X, Y die oben angegebene Bedeutung haben,
sind neu.

4. Sie werden hergestellt, indem man Verbindungen der Formel VI

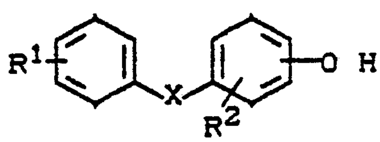

VI

in welcher
$R^1$, $R^2$, X die oben angegebene Bedeutung haben,
mit Verbindungen der Formeln VII oder VIII

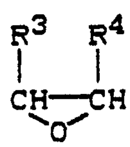

VII

in welcher
$R^3$ und $R^4$ die oben angegebene Bedeutung haben,

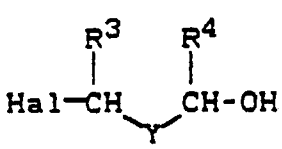

VIII

in welcher
Hal, $R^3$, $R^4$, Y die oben angegebene Bedeutung haben,
umsetzt.

Die Verbindungen der Formel I sowie ihre Salze mit Säuren eignen sich zur Bekämpfung von Insekten. Sie lassen sich besonders bevorzugt zur Bekämpfung von Flöhen einsetzen, insbesondere gegen Pulex irritans, Xenopsylla cheopis, Ctenocephalides canis, Ctenocephalides felis, Ceratophyllus gallinae, Echidnophaga gallinacea, Tunga penetrans. Die Verbindungen der Formel I besitzen die Entwicklung von Insekten hemmende Eigenschaften. Sie können daher gegen alle oder einzelne Entwicklungsstadien von Insekten, insbesondere der Flöhe, eingesetzt werden.

Bevorzugt sind Verbindungen der Formel I,
in welcher
X für O, S, $CH_2$ steht,
Y für geradkettiges oder verzweigtes Alkylen mit bis zu 12 C-Atomen, das gegebenenfalls durch 1 bis 6 Sauerstoffatome unterbrochen ist, steht.
$R^1$ für gleiche oder verschiedene Reste aus der Gruppe Wasserstoff, Halogen wie Chlor steht,
$R^2$ für gleiche oder verschiedene Reste aus der Gruppe Wasserstoff, Chlor, Brom, $C_{1-4}$-Alkyl wie Methyl, Ethyl, i-Propyl, t-Butyl, $C_{2-4}$-Alkenyl wie Allyl, $C_{1-4}$-Halogenalkyl wie Trifluormethyl, $C_{1-4}$-Alkoxy, $C_{1-4}$-Halogenalkoxy wie Trifluormethoxy, steht,
$R^3$ für Wasserstoff, $C_{1-4}$-Alkyl wie Methyl, Ethyl steht,
$R^4$ für Wasserstoff, $C_{1-4}$-Alkyl wie Methyl, Ethyl, Propyl, Butyl, $C_{1-4}$-Halogenalkyl wie Trifluormethyl, $C_{1-4}$-Alkoxy-$C_{1-4}$-alkyl wie Methoxymethyl, n- oder t-Butoxymethyl, i-Propoxymethyl, $C_{2-4}$-Alkenyl wie Allyl, $C_{2-4}$-Alkenoxy-$C_{1-4}$-alkyl wie Alkyloxymethyl steht,
$R^3$ und $R^4$ gemeinsam für eine direkte Bindung stehen und gemeinsam mit den angrenzenden C-Atomen und Y für Cyclopentyl oder Cyclohexyl stehen können,
$R^5$ für Halogen wie Chlor, Brom, $C_{1-4}$-Alkyl wie Methyl, Ethyl, $C_{1-4}$-Halogenalkyl wie Trifluormethyl, Alkoxy wie Methoxy, Amino, Acylamino wie z.B. substituiertes Phenylureido, Morpholinyl, Dimethylmorpholinyl, Piperazinyl, N-Alkylpiperazinyl, steht.

Besonders bevorzugt sind Verbindungen der Formel I,
in welcher
X für O steht,
Y für Alkylen mit bis zu 5 C-Atomen, das gegebenenfalls durch O unterbrochen ist wie z.B. Methylen $-CH_2-O-CH_2-$, $-CH_2-O-CH_2-O-CH_2-$, $-CH(CH_3)-O-CH_2-$.
$R^1$ für Wasserstoff steht,
$R^2$ für Wasserstoff, Halogen, insbesondere Chlor oder Brom, $C_{1-4}$-Alkyl, $C_{2-4}$-Alkenyl, insbesondere Alkyl steht,
$R^3$ für Wasserstoff steht,
$R^4$ für Wasserstoff, $C_{1-6}$-Alkyl, insbesondere Methyl, Ethyl, Propyl, Butyl, $C_{2-4}$-Alkenyl, insbesondere Allyl, $C_{1-4}$-Alkoxy-$C_{1-4}$-alkyl, insbesondere Methoxy-, Ethoxy-, Isopropoxy-, Butoxymethyl, $C_{2-4}$-Alkenoxy-$C_{1-4}$-alkyl, insbesondere Allyloxymethyl steht,
$R^3$ und $R^4$ für eine direkte Bindung stehen und gemeinsam mit den angrenzenden C-Atomen und Y für Cyclopentyl oder Cyclohexyl stehen,
$R^5$ für $C_{1-4}$-Alkyl, $C_{1-4}$-Alkoxy, insbesondere Methoxy, $C_{1-4}$-Halogenalkyl, insbesondere Trifluormethyl, Amino, Halogen, insbesondere Chlor, $C_{1-4}$-Alkylureido, insbesondere Methylureido, Phenylureido, wobei der Phenylring gegebenenfalls durch Halogen, Alkyl, Alkoxy, Halogenalkyl, Halogenalkoxy, substituiert sein kann, steht.

Im einzelnen seien genannt:

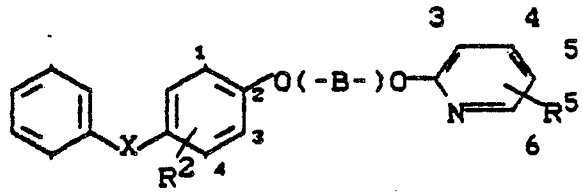

| X | $R^2$ | B | $R^5$ |
|---|---|---|---|
| O | 1,3 $Cl_2$ | $-CH_2-CH_2-O-CH_2-\underset{CH_3}{CH}-$ | 3-$NH_2$ |
| S | H | $-CH_2-\overset{CH_3}{CH}-O-CH_2-\underset{CH_3}{CH}-$ | 5-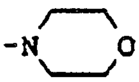 |
| O | 1,3-$Cl_2$ | $-CH_2-CH_2-O-CH_2CH_2-$ | 4-$NH_2$ |
| O | 1,3-$Cl_2$ | $-CH_2-CH_2-O-CH_2CH_2-$ | 3-$OCH_3$ |
| S | 1,3-$Cl_2$ | $-CH_2-CH_2-O-CH_2CH_2-$ | 5-$OCH_3$ |
| O | 3-Br | $-CH_2-CH_2-O-CH_2CH_2-$ | 5-$OCH_3$ |

| X | $R^2$ | B | $R_5$ |
|---|---|---|---|
| $CH_2$ | 1,3-$Cl_2$ | $-CH_2-CH_2-O-CH_2CH_2-O-CH_2CH_2-$ | 3-$NH_2$ |
| $CH_2$ | H | 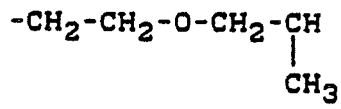 | 5-$CF_3$ |
| O | H | 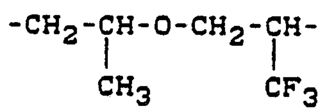 | 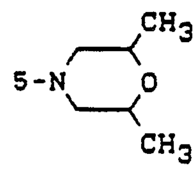 |
| S | H | 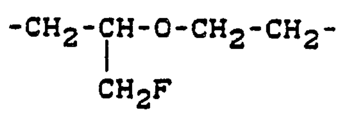 | 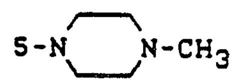 |
| O | 1,3-$Cl_2$ | $-CH_2-CH_2-O-CH_2CH_2-$ | 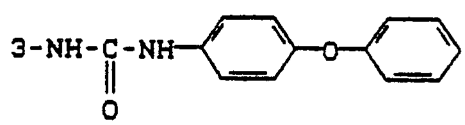 |
| O | 1,3-$Cl_2$ | 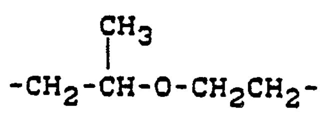 | 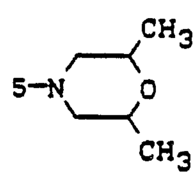 |
| S | 1,3-$Cl_2$ | 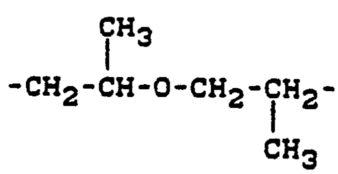 | 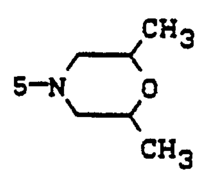 |

| X | $R^2$ | B | $R^5$ |
|---|---|---|---|
| $CH_2$ | 1,3-$Cl_2$ | $-CH_2-CH(CH_3)-O-CH_2CH_2-$ | 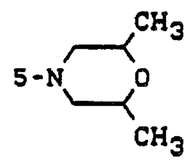 |
| O | H | $-CH_2-CH_2-O-CH_2CH_2-$ | 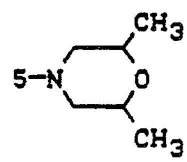 |
| O | 1,3-$Cl_2$ | $-CH_2CH_2O-CH_2-CH_2-$ | 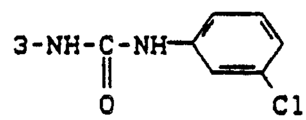 |
| $-CH(CH_3)-$ | H | $-CH_2CH_2-O-CH_2CH_2-$ | 4-$OCH_3$ |
| O | H | 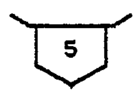 | 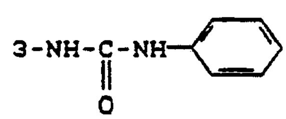 |
| O | H | $-CH_2CH_2-O-CH_2CH_2CH_2-$ | 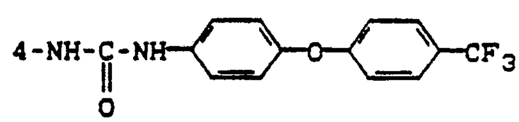 |

Setzt man bei Verfahren 2a zur Herstellung der erfindungsgemäßen Verbindungen als Verbindung der Formel II 2-[2-(4′-Phenoxyphenoxy)-ethoxy]-ethanol und als Verbindung der Formel III 3-Chlorpyridin ein, läßt sich der Reaktionsverlauf durch folgendes Formelschema kennzeichnen:

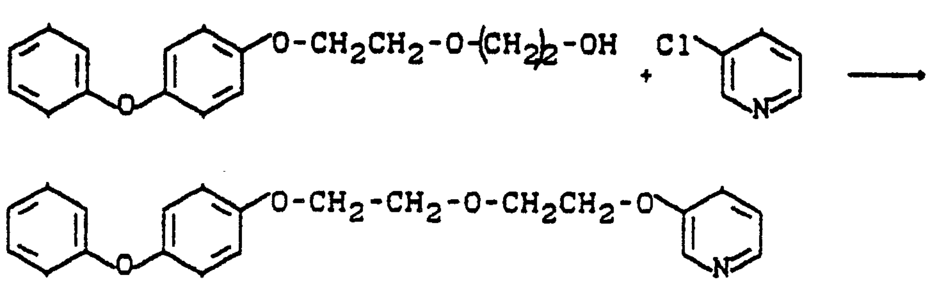

Bevorzugt werden Verbindungen der Formeln II und III eingesetzt, in denen $R^1$, $R^2$, $R^3$, $R^4$, $R^5$, X, Y, die bei den Verbindungen der Formel I angegebenen bevorzugten oder besonders bevorzugten Bedeutungen besitzen.

Im einzelnen seien folgende Verbindungen der Formel II genannt:

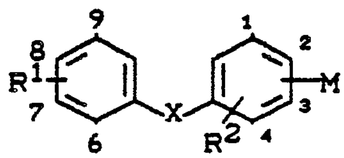

| $R^1$ | X | $R^2$ | M |
|---|---|---|---|
| H | $CH_2$ | 1-Cl | $3-O-CH_2-CH_2-O-CH_2CH_2-OH$ |
| 7-Cl | O | H | $2-O-CH_2-CH_2-O-CH_2CH_2-OH$ |
| 7-Cl | O | H | $2-O-CH_2-CH_2-O-CH_2CH_2-O-CH_2CH_2-OH$ |
| 7-Cl | S | H | $2-O-CH_2-CH_2-O-CH_2CH_2-OH$ |
| H | O | $3-OCH_3$ | $2-O-CH_2-CH-O-CH_2-CH-OH$<br>$CH_3$ $CH_3$ |
| H | O | $3-OCH_3$ | $2-O-CH_2-CH-O-CH_2-CH_2-OH$<br>$CF_3$ |
| 7-Cl | $CH-CH_3$ | H | $2-O-CH_2-CH_2-O-CH_2CH_2-OH$ |
| 7-Cl | O | H | $2-O-CH_2-CH-O-CH_2CH_2-OH$<br>$CH_2F$ |
| 7-Cl | O | H | $3-O-CH_2-CH-O-CH_2CH_2-OH$<br>$CH_2F$ |
| H | S | $3-OCH_3$ | $2-O-CH_2-CH-O-CH_2CH_2-OH$<br>$CH_2F$ |
| 7-Cl | O | H | $3-O-CH_2-CH-O-CH_2CH_2-OH$<br>$CH_2-OCF_3$ |
| 7-Cl | S | H | $2-O-CH_2-CH-O-CH_2CH_2-OH$<br>$CH_2-OCF_3$ |
| H | O | 1-Cl,3-t-Butyl | $2-O-CH-CH_2-O-CH_2-CH-OH$<br>$CH_3$ $CH_3$ |

In einzelnen seien folgende Verbindungen der Formel III genannt: 2-Chlorpyridin, 2-Brompyridin, 3-Chlorpyridin, 3-Brompyridin, 2-Chlor-5-trifluormethylpyridin, 2-Brom-5-methylpyridin, 4-Chlorpyridin, 2-Chlor-6-methylpyridin, 2,3-Dichlorpyridin, 2,5-Dichlorpyridin, 2,6-Dichlorpyridin, 2,6-Dibrompyridin, 3,5-Dichlorpyridin, 2,6-Difluorpyridin.

Die Verbindungen der Formel II sind neu, ihre Herstellung wird weiter unten beschrieben. Die

Verbindungen der Formel III sind bekannt.

Die Umsetzung erfolgt durch Erhitzen der Verbindungen II und III in Gegenwart von Verdünnungsmitteln, Basen sowie gegebenenfalls Katalysatoren.

Die Umsetzung erfolgt bei Temperaturen von 0 - 200 °C, bevorzugt bei 20 - 100 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methyl isopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Tris-[2-(2-methoxyethoxy)]-ethyl-amin (TDA-1), Benzyltriethylammoniumchlorid (TEBA), 1,4,7,10,13,16-Hexaoxacyclooctadecan (18-Crown-6), Tetrabutylammoniumbromid, Tetrabutylammoniumhydrogensulfat, Tetrabutylammoniumjodid, Methyltrialkyl ($C_8$-$C_{10}$)-ammoniumchlorid (Adogen 464).

Die Verbindungen der Formeln II und III werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 2b zur Herstellung der Verbindungen der Formel I als Verbindung der Formel IV 3-Chlorpropyloxy-diphenylether und als Verbindung der Formel V 4-Hydroxy-3-amino-pyridin ein, läßt sich der Reaktionsverlauf durch folgendes Formelschema wiedergeben:

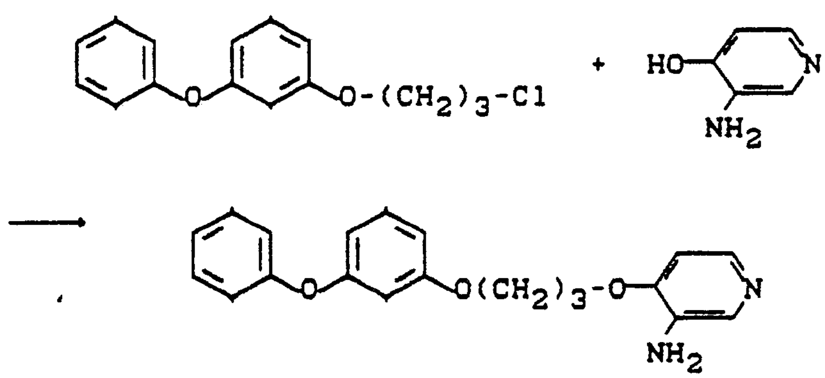

Das Verfahren wird bevorzugt angewendet, wenn die Verbindung der Formel V eine Aminogruppe enthält.

Bevorzugt werden Verbindungen der Formeln IV und V eingesetzt, in denen $R^1$, $R^2$, $R^3$, $R^4$, $R^5$, X, Y, die bei den Verbindungen der Formel I angegebenen bevorzugten oder besonders bevorzugten Bedeutungen besitzen.

Im einzelnen seien folgende Verbindungen der Formel IV genannt:

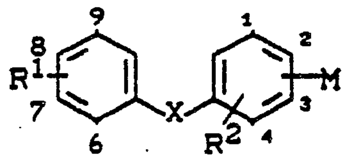

| $R^1$ | X | $R^2$ | M |
|---|---|---|---|
| H | $CH_2$ | 1-Cl | 3 - $O-CH_2-CH_2-Cl$ |
| 7-Cl | O | H | 2-$O-CH_2CH_2-Cl$ |
| H | S | H | 3-$O-CH_2CH_2-Cl$ |
| 7-Cl | O | H | 2-$O-CH_2CH_2CH_2-Cl$ |
| 7-Cl | O | H | 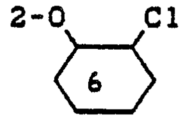 |
| 7-Cl | O | H | 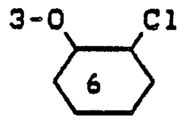 |
| H | O | H | 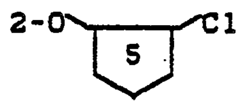 |
| H | $CH_2$ | H | 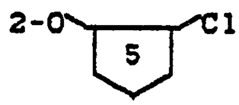 |

| $R^1$ | X | $R^2$ | M |
|---|---|---|---|
| H | O | H | $2\text{-}O\text{-}CH_2CH_2CH_2\text{-}Br$ |
| H | O | H | $2\text{-}O\text{-}CH_2\text{-}CH(CH_3)\text{-}Cl$ |
| 7-Cl | O | H | $2\text{-}O\text{-}CH_2\text{-}CH(CH_3)\text{-}Cl$ |
| 7-Cl | O | H | $2\text{-}O\text{-}CH_2\text{-}CH(C_2H_5)\text{-}Cl$ |
| H | O | H | $2\text{-}O\text{-}CH_2CH_2\text{-}O\text{-}CH_2CH_2\text{-}Cl$ |
| H | O | H | $2\text{-}O\text{-}CH_2CH_2\text{-}O\text{-}CH_2CH_2\text{-}OCH_2CH_2\text{-}Cl$ |

In einzelnen seien folgende Verbindungen der Formel V genannt: 2-Hydroxypyridin, 2-Mercaptopyridin, 2-Hydroxypyrimidin, 3-Hydroxypyridin, 2-Amino-3-hydroxypyridin, 2-Chlor-3-hydroxypyridin, 2,3-Dihydroxypyridin.

Die Verbindungen der Formel IV sind neu, ihre Herstellung wird weiter unten beschrieben. Die Verbindungen der Formel V sind bekannt.

Die Umsetzung erfolgt durch Erhitzen der Verbindungen IV und V in Gegenwart von Verdünnungsmitteln, Basen sowie gegebenenfalls Katalysatoren.

Die Umsetzung erfolgt bei Temperaturen von 0 - 200 °C, bevorzugt bei 20 - 100 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Methyltrialkyl ($C_8$-$C_{10}$)-ammoniumchlorid (Adogen 464), 1,4,7,10,13,16-Hexaoxacyclooctadecan (18-Crown-6), Tetrabutylammoniumbromid, Tris-[2-(2-methoxyaethoxy)-aethyl]-amin (TDA-1), Benzyltriethylammoniumchlorid (TEBA).

Die Verbindungen der Formeln IV und V werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I

in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 4 zur Herstellung der Verbindungen der Formel II als Verbindung der Formel VI 3-Hydroxy-diphenylether ein und als Verbindung der Formel VII Propylenoxid, so läßt sich der Reaktionsablauf durch folgendes Formelschema darstellen:

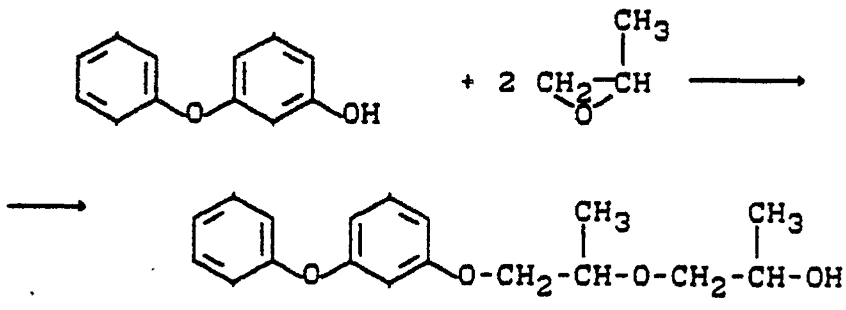

Bevorzugt werden Verbindungen der Formeln VI und VII eingesetzt, in welchen $R^1$, $R^2$, $R^3$, $R^4$, X, Y die bei den Verbindungen der Formel I angegebenen bevorzugten Bedeutungen besitzen.

Im einzelnen seien folgende Verbindungen der Formel VI genannt:
2-Hydroxy-diphenylether, 3-Hydroxy-diphenylether, 4-Hydroxy-diphenylether, 2-Hydroxy-diphenylmethan, 3-Hydroxy-diphenylmethan, 4-Hydroxy-diphenylmethan, 3′-Trifluormethyl-4-hydroxy-diphenylmethan, 3′-Trifluormethyl-4-hydroxy-diphenylether, 4′-Fluor-4-hydroxy-diphenylether, 4′-Chlor-4-hydroxy-diphenylether, 4′-Chlor-3-hydroxy-dophenylether, 4′-Methoxy-4-hydroxy-diphenylether, 4′-Chlor-3′-trifluormethyl-4-hydroxy-diphenylether, 4-Hydroxy-diphenylamin, 4′-Methyl-4-hydroxy-diphenylether, 4-Hydroxy-diphenylsulfid, 4-(2-Phenyl-2-propyl)-phenol.

Im einzelnen seien folgende Verbindungen der Formel VII genannt:
Propylenoxid, 1-Chlor-2,3-epoxypropan, 1-Fluor-2,3-epoxypropan, 3,3,3-Trifluoro-1,2-epoxypropan, Butyl-2,3-epoxypropylether, 2,3-Epoxypropyl-isopropylether, Allylglycidylether, 1-Brom-2,3-epoxypropan, 1,3-Butadienmonoxid, Cyclopentenoxid, Cyclohexenoxid, Cycloheptenoxid, 1,2-Hexenoxid, 1,2-Decenoxid, 1,2-Dodecenoxid, 1,2-Hexadecenoxid, 2,2-Dimethyl-1-methoxyaethylenoxid, 1,2-Epoxy-3-methoxypropan, 1,5-Hexadienmonoxid, tert.-Butyl-2,3-epoxypropylether, 3-Methoxy-2,2-dimethylenpoxypropan, 1,2-Butylenoxid, 2,2-Dimethyloxiran, 2,3-Dimethyloxiran, Ethylenoxid.

Die Verbindungen der Formeln VI und VII sind bekannt.

Die Umsetzung erfolgt durch Erhitzen einer Mischung der Ausgangsverbindungen in einem Verdünnungsmittel und Waser in Gegenwart von Basen sowie gegebenenfalls in Gegenwart eines Katalysators.

Die Umsetzung erfolgt bei Temperaturen von 0 bis 200 °C, insbesondere bei 50 - 150 °C.

Als Verdünnungsmittel seien genannt:
Pentan, Hexan, Heptan, Cyclohexan, Petrolether, Benzin, Ligroin, Benzol, Toluol, Xylol, Diethylether, Dibutylether, Diglykoldimethylether, Dioxan, Dimethylsulfoxid, Wasser.

Als Basen seien genannt:
Alkali- und Erdalkalihydroxide, -carbonate.

Als Katalysatoren seien genannt:
Tetrabutylammoniumbromid, Tetrabutylammoniumhydrogensulfat, Tetrabuylammoniumjodid, Triethylbenzylammoniumchlorid (TEBA), Methyl ($C_8$-$C_{10}$)-ammoniumchlorid (Adogen 464), Hexadecyl-tributylphosphoniumbromid, Tetraethylammoniumjodid.

Pro Mol. der Verbindung VI werden mindestens 2 Mol. Verbindung der Formel VII eingesetzt.

Nach erfolgter Umsetzung erfolgt die Aufarbeitung wie folgt:
Die organische Phase wird von der Wasserphase abgetrennt; nach dem Trocknen über z.B. Natriumsulfat wird vom org.Verdünnungsmittel abdestilliert, aus dem Rückstand werden die Verbindungen der Formel II in üblicher Weise z.B. durch Destillation gereinigt.

Die Umsetzung der Verbindungen der Formel VI mit Verbindungen der Formel VIII erfolgt durch Erhitzen einer Mischung der Ausgangsverbindungen in einem Verdünnungsmittel und Wasser in Gegenwart von Basen und Katalysatoren.

Diese Art der Umsetzung wird bevorzugt gewählt, um zu Verbindungen der Formel I zu gelangen, in denen $R^3$ für andere Reste als Wasserstoff steht.

Die Umsetzung erfolgt bei Temperaturen von 0 bis 200 °C, insbesondere bei 50 - 150 °C.

Als Verdünnungsmittel seien genannt:

Pentan, Hexan, Heptan, Cyclohexan, Petrolether, Benzin, Ligroin, Benzol, Toluol, Xylol, Diethylether, Dibutylether, Diglykoldimethylether, Dioxan, Dimethylsulfoxid.

Als Basen seien genannt:

Alkali- und Erdalkalihydroxide-, carbonate.

Als Katalysatoren seien genannt:

Tetrabutylammoniumbromid, Tetrabutylammoniumhydrogensulfat, Tetrabutylammoniumjodid, Triethylbenzylammoniumchlorid (TEBA), Methyl ($C_8$-$C_{10}$)-ammoniumchlorid (Adogen 464), Hexadecyl-tributylphosphoniumbromid, Tetraethylammoniumjodid.

Die Verbindungen VI und VIII werden in äquimolarem Verhältnis zueinander eingesetzt.

Nach erfolgter Umsetzung erfolgt die Aufarbeitung wie folgt:

Die organische Phase wird von der Wasserphase abgetrennt; nach dem Trocknen über z.B. Natriumsulfat wird vom organischen Verdünnungsmittel abdestilliert, aus dem Rückstand werden die Verbindungen der Formel II in üblicher Weise z.B. durch Destillation gereinigt.

Wie bereits erwähnt, sind die erfindungsgemäßen Wirkstoffe insektizid, insbesondere gegen Flöhe wirksam. Sie werden zur Bekämpfung von Flöhen bei Haustieren wie Hunden oder Katzen sowie der Umgebung der Haustiere eingesetzt.

Die Anwendung der Wirkstoffe erfolgt direkt oder in Form von geeigneten Zubereitungen, dermal, enteral oder parenteral, oder durch Behandlung der Umgebung oder mit Hilfe wirkstoffhaltiger Formkörper wie z.B. Bänder, Halsbänder, Gliedmaßenbänder, Markierungsvorrichtungen.

Die dermale Anwendung geschieht z.B. in Form des Tauchens (Dippen), Sprühens (Sprayen) oder Aufgießens (pour-on und spot-on) und des Einpuderns.

Zubereitungen zur dermalen Anwendung sind z.B. Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper.

Lösungen, Suspensions- und Emulsionskonzentrate sowie Mikroemulsionen die vor der Anwendung mit Wasser verdünnt werden, Pulver, Stäube und Aerosolformulierungen werden zur Behandlung der Umgebung der Haustiere eingesetzt.

Diese Zubereitungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden.

Zu den flüssigen Verdünnungsmitteln zählen neben Wasser Alkohole wie Methanol, Ethanol, Isopropanol, n-Butanol, Amylalkohol, Octanol;

Glykole wie Propylenglykol, 1,3-Butylenglykol, Ethylglykol, Dipropylenglykolmonomethylether;

Glycerin;

aromatische Alkohole wie Benzylalkohol;

Carbonsäureester wie z.B. Ethylacetat, Benzylbenzoat, Butylacetat, Propylencarbonat, Milchsäureethylester;

aliphatische Kohlenwasserstoffe wie Paraffine, Cyclohexan, Methylenchlorid, Ethylenchlorid;

aromatische Kohlenwasserstoffe wie Xylol, Toluol, Alkylnaphthaline, Chlorbenzole;

Ketone wie z.B. Aceton und Methylethylketon, Methylisobutylketon, Cyclohexanon;

natürliche und synthetische Mono- und Triglyceride mit natürlichen Fettsäuren wie Baumwollsaatöl, Erdnußöl, Maiskeimöl, Olivenöl, Ricinusöl, Sesamöl;

weiterhin Dimethylsulfoxid, Dimethylacetamid, Dimethylformamid, N-Methylpyrrolidon, Dioxan, 2,2-Dimethyl-4-oxymethyl-1,3-dioxolan.

Zu den oberflächenaktiven Stoffen zählen:

Emulgatoren und Netzmittel wie anionaktive Tenside, z.B. Alkylsulfonate, Alkylsulfate, Arylsulfonate, Na-Laurylsulfate, Fettalkoholethersulfate, Mono/Dialkylpolyglykoletherorthophosphorsäureester-Monoethanolaminsalz, Calciumalkylarylsulfonat;

kationaktive Tenside, z.B. Cetyltrimethylammoniumchlorid;

ampholytische Tenside, z.B. Di-Na-N-lauryl-betaiminodipropionat oder Lecithin;

nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:

Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;
Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere;
Fettalkohole wie Isotridecylalkohol, 2-Octyldodecanol, Cetylstearylalkohol, Oleylalkohol.

Zur Herstellung von Pulvern und Stäuben wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Diatomeenerde, Kochsalz, Aluminiumoxide, Kieselsäuren, Tonerden, gefälltes oder kolloidales Siliciumdioxid. Organische Stoffe sind Laktose, Stärke und Cellulose bzw. Cellulosederivate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben. Als solche seien wirkstoffhaltige Formkörper wie z.B. Platten, Bänder, Streifen, Halsbänder, Gliedmaßenbänder, Markierungsvorrichtungen genannt.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtspronzent Wirkstoff.

Formulierungen die erst nach weiterer Verdünnung angewendet werden enthalten 1 - 95 Gewichtsprozent, bevorzugt 5 - 90 Gewichtsprozent Wirkstoff.

Formulierungsbeispiele

| 1. Herstellung eines Emulsionskonzentrates | |
|---|---|
| a) Wirkstoff gemäß Beispiel 1 (100 %) | 25,00 g |
| nichtionischer Emulgator (Emulgator 368® = Alkylarylpolyglycolether MG ca. 1165) | 25,00 g |
| Dipropylenglykolmonomethylether ad | 100,00 ml |

Herstellung

Die Substanzen werden zusammen gewogen und so lange gerührt, bis eine klare Lösung entstanden ist.

Vor Gebrauch wird die Lösung auf ihre Anwendungskonzentration verdünnt.

| | |
|---|---|
| b) Wirkstoff gemäß Beispiel 2 (100 %)<br>Polyoxyethylenstearat<br>Sorbitan-Sesquioleat<br>Wasser | 1,00 g<br>0,50 g<br>0,40 g<br>4,00 g |
| Polyethylenglykol 200 ad 100 ml | |

Herstellung und Anwendung wie bei 1a

| 2. Herstellung einer pour-on-Formulierung | |
|---|---|
| a) Zusammensetzung Wirkstoff gemäß Beispiel 1 (100 %)<br>Isopropylmyristat<br>2-Octyldodecanol | 5,00 g<br>30,00 g<br>20,00 g |
| Isopropanol ad 100 ml | |

Herstellung:

Die Substanzen werden zusammen gewogen und bis zur klaren Lösung gerührt.

| | |
|---|---|
| b) Zusammensetzung Wirkstoff gemäß Beispiel 1 (100 %)<br>Silikonöl 100 | 0,50 g<br>30,00 g |
| Butylacetat ad 100 ml | |

Herstellung wie bei Beispiel a)

| 3. Herstellung einer Mikroemulsion | |
|---|---|
| Wirkstoff gemäß Beispiel 1 (100 %)<br>Eumulgin B3®(Alkylarylpolyglycolether)<br>Cetiol HE® (Polyolfettsäureester)<br>Isopropylmyristat<br>Benzylalkohol | 13,00 g<br>30,00 g<br>30,00 g<br>5,00 g<br>3,00 g |
| Wasser ad 100 ml | |

Herstellung:

Der Wirkstoff wird in den lipophilen Komponenten (Eumulgin, Cetiol, Benzylalkohol, Isopropylmyristat) dispergiert.

Nach Erwärmen auf 60°C wird Wasser der gleichen Temperatur zugemischt und abgekühlt. Die entstandene Mikroemulsion gleicht außerlich einer klaren Lösung.

| 4. Herstellung einer Sprühformulierung | |
|---|---|
| Zusammensetzung | |
| Wirkstoff gemäß Beispiel 1 (100 %) | 20 g |
| Emulgator Toximul® (Mischung aus Alkylbenzolsulfonat-Ca und nichtionogenen Emulgatoren und Methanol) | 7 g |
| Emulgator Toximul S® (Mischung aus Alkylbenzolsulfonat-Ca und nichtionogenem Emulgator und Methanol) | 5 g |
| Sovesso 200® (Alkylnaphthalin-Gemisch hochsiedender Erdölfraktionen) | ad 100 ml |

Herstellung:

Der Wirkstoff wird mit den restlichen Komponenten zusammen gewogen, gerührt und vor Anwendung mit Wasser auf die Anwendungskonzentration verdünnt.

Anwendungsbeispiele

A. In vitro-Test an Flöhen (alle Entwicklungsstadien)

Testobjekt: Alle Stadien (Eier, Larven, Puppen, Adulte) von Ctenocephalides felis

Testverfahren

Die zu testende Substanz wird in der gewünschten Konzentration homogen in Blutmehl verteilt. In dieses Gemisch aus Blutmehl und Testsubstanz werden Floheier, die von flohbefallenen Katzen gesammelt wurden, gebracht und bei 80 % rel. Feuchte und 25° C inkubiert.

Nach Ablauf der Entwicklungszeit von 3 bis 4 Wochen wird festgestellt, ob adulte Flöhe zur Entwicklung gekommen sind. Dabei bedeutet eine 100 %ige Wirkung, daß keine adulten Flöhe festgestellt wurden und 0 %, daß lebende adulte Flöhe festgestellt wurden.

Der Wirkstoff gemäß Beispiel 3 zeigt bei 5 ppm 100 % Wirkung.

B. In vivo-Test an Flöhen (alle Entwicklungsstadien)

Testobjekt: Eier, Larven, Puppen von Ctenocephalides felis in der Umgebung von Hunden/Katzen, adulte Flöhe auf Hunden/Katzen.

Testverfahren

Läger von flohfreien Hunden/Katzen in Boxen werden mit der zu testenden Substanz in der gewünschten Anwendungskonzentration besprüht. Zu bestimmten Zeiten nach der Applikation werden Eier von Ctenocephalides felis, die von behandelten Katzen gesammelt wurden, auf die Läger der Hunde/Katzen gebracht. Es wird festgestellt, ob und zu welchem Zeitpunkt die in den Boxen befindlichen Hunde/Katzen von adulten Flöhen befallen werden.

Dabei bedeutet eine 100 %ige Wirkung, daß Hunde/Katzen keinen Flohbefall aufweisen und 0 % Wirkung, daß Hunde/Katzen von Flöhen befallen sind.

Der Wirkstoff gemäß Beispiel 3 zeigt 100 % Wirkung.

Herstellungsbeispiele

Verfahren 2a:

Allgemeine Vorschrift:

Ein Gemisch aus 1 m Verbindung der Formel II und der etwa 1,1- bis 3-fachen molaren Menge Verbindung der Formel III, der etwa 6-fachen molaren Menge Na-OH-Pulver, der etwa 1,1-fachen Menge $K_2CO_3$-Pulver sowie der etwa 0,02-fachen Menge des Phasentransferkatalysators Tris-(2-(2-methoxyethoxy)-ethyl)-amin wird in Toluol ca. 36 Stunden am Rückfluß gekocht. Nach dem Abkühlen wird die Toluolphase mit Wasser gewaschen, über $Na_2SO_4$ getrocknet und im Vakuum eingeengt. Das entstandene Produkt wird durch Kugelrohrdestillation weiter gereinigt. Gemäß dieser Vorschrift werden erhalten:

Beispiel 1

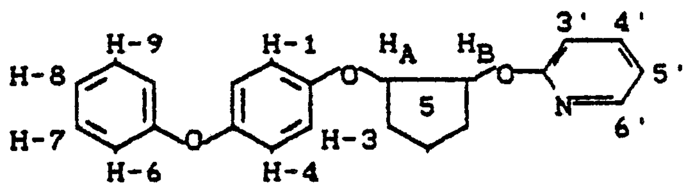

$^1$H-NMR, DMSO-$d_6$: 8,18 ppm (m, H-6'); 7,71 ppm (m, H-4') 5,40 ppm (m, 1H, $H_B$); 4,81 ppm (m, 1H, $H_A$); 2,21-1.72 ppm (m, 6H); 7,37-6,81 ppm (m, 11H, 9 aromat.H, + H-3' + H-5').

Beispiel 2

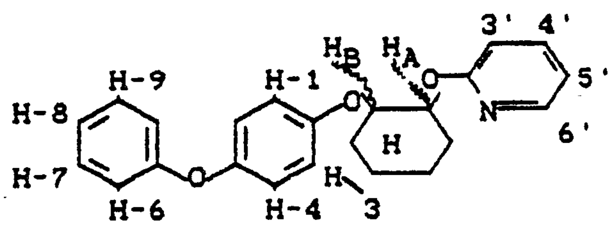

$^1$H-NMR, DMSO-$d_6$:
8,16 pp (m, H-6'); 7,37-6,90 ppm (m, 10H, 9 aromat. H und H-5'); 7,68-7,62 ppm (m, H-4'); 6,67 (d J, 8,3 Hz, H-3'); 4,46-4,39 ppm (m, $H_A$); 5,27-5,20 ppm (m, $H_B$); 2,11 ppm (m, 2H); 1,7 ppm (m, 2H); 1,52-1,40 ppm (m, 4H).

Beispiel 3

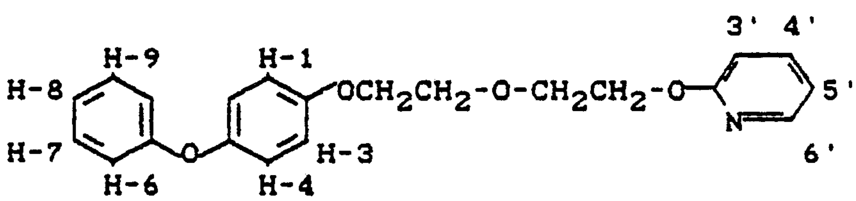

$^1$H-NMR, DMSO-$d_6$:
8,15 ppm (m, H-6'); 7,73-4,67 ppm (m, H-4'); 7,37-6,90 ppm (m, 10H, 9 aromat. H + H-5'); 6,82 ppm (m, H-3'); 4,41-4,48 ppm (m, 2H); 4,11-4,08 ppm (m, 2H); 3,83-3,79 ppm (m, 4H).

Beispiel 4

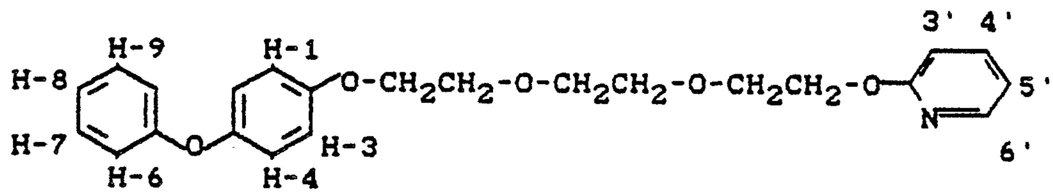

$^1$H-NMR, DMSO-$d_6$:
8,16-8,13 ppm (m, H-6'); 7,72-7,66 ppm (m, H-4'); 7,37-6,90 ppm (m, 10H, 9 aromat. H + H-5'); 6,83-6,8 ppm (m, H-3'); 4,39-4,35 ppm (m, 2H); 4,08-4,05 ppm (m, 2H); 3,77-3,73 ppm (m, 4H); 3,61 ppm (m, 4H).

Verfahren 2b:

Allgemeine Vorschrift:

Ein Gemisch aus ca. 20 mmol Verbindung der Formel IV und der etwa 1,5- bis 2-fachen molaren Menge Verbindung der Formel V, der etwa 0,05-fachen Menge des Phasentransferkatalysators (Adogen 464) wird in 30 ml 45 %iger NaOH und 30 ml Methylenchlorid 18 Stunden bei 50 bis 70 °C gerührt. Nach dem Abkühlen wird mit 0,5 l Wasser verdünnt, dreimal mit jeweils 50 ml Methylenchlorid extrahiert, die Methylenchloridphasen eingeengt und der verbleibende Rückstand aus Diethylether umkristallisiert. Gemäß dieser Vorschrift wird erhalten:

Beispiel 5

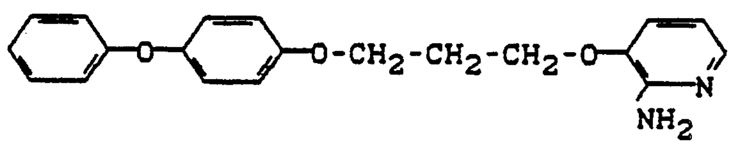

gelbe Kristalle, Fp 47 - 48 °C

Beispiel 6

4,2 mmol (1,4 g) der Verbindung des Beispiels 5 werden in 80 ml trockenem Aceton mit 6,4 mmol (1,2 g) 3,4-Dichlorphenylisocyanat 18 Stunden am Rückfluß gekocht. Das Lösungsmittel wird unter vermindertem Druck abdestilliert und der Rückstand aus Cyclohexan umkristallisiert. Ausbeute 2,0 g (ca. 92 %).

Beispiel 7

3,6 mmol (1,2 g) der Verbindung des Beispiels 5 werden in 80 ml trockenem Acetonitril mit 5,7 mmol (1,2 g) 4-Phenoxyphenylisocyanat 18 Stunden am Rückfluß gekocht. Nach Abdestillieren des Lösungsmittels im Vakuum wird der Rückstand aus Cyclohexan umkristallisiert. Ausbeute 1,4 g (ca. 72 %).

Herstellung der Ausgangsverbindungen der Formel II gemäß Verfahren 4:

Pro mol Verbindung der Formel VI werden 3 bis 5 mol Epoxid der Formel VII in Gegenwart von 2 bis 3 mol NaOH, 0,005 bis 0,02 mol Phasentransferkatalysator Tetrabutylammoniumbromid in einer Mischung aus

1 l Toluol und ca. 340 ml Wasser bei 50 bis 130 °C 48 Stunden gerührt. Nach dem Abkühlen wird das Gemisch mit gesättigter NaCl-Lösung versetzt, und die Toluolphase abgetrennt. Toluol wird im Vakuum abdestilliert und der Rückstand destilliert. Das Rohprodukt der Umsetzung kann aber auch ohne vorherige Reinigung sofort weiter umgesetzt werden.

Gemäß dieser Vorschrift werden erhalten:

Beispiel a)

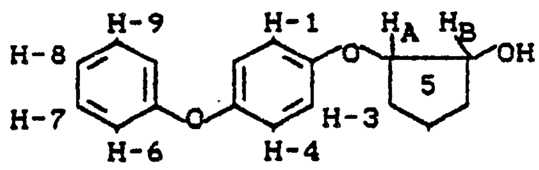

$^1$H-NMR (DMSO-$d_6$):
7,37-6,91 ppm (m, 9 aromat. H); 5,05 ppm (S, OH); 4,43-4,41 ppm und 4,07 ppm (m, 2H, $H_A$ und $H_B$); 2,13-1,27 (m, 6H, Cyclopentanring).

Beispiel b)

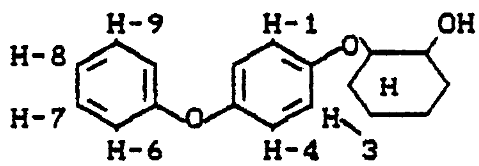

$^1$H-NMR (DMSO-$d_6$):
7,37-6,90 ppm (m, 9 aromat. H); 4,91 ppm (d, J = 4,6 Hz, OH); 4,01-3,94 ppm (m, $H_A$ oder $H_B$); 3,56-3,49 ppm (m, $H_A$ oder $H_B$); 1,99-1,26 ppm (m, 8H).

Beispiel c)

14 g 4-Hydroxydiphenylether wird mit 25 g 2-(2-Chlorethoxy)-ethanol in Gegenwart von 30 g $K_2CO_3$ und 4 g KJ in 250 ml Acetonitril ca. 24 Stunden am Rückfluß erhitzt. Nach dem Abkühlen wird vom Lösungsmittel abdestilliert, der verbleibende Rückstand mit Natronlauge und Toluol gewaschen und anschließend die Toluolphase über $Na_2SO_4$ getrocknet. Es wird erneut vom Lösungsmittel abdestilliert. Man erhält gemäß dieser Vorschrift:

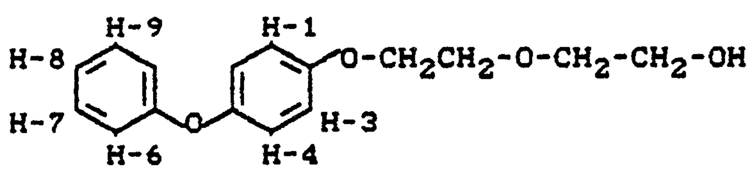

Siedepunkt: 250 °C / 0,2 mbar.
Ausbeute: 9,8 g (48 % der Theorie)

Beispiel d)

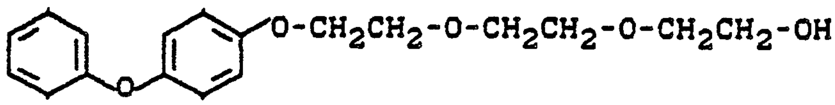

Ausbeute: 6,0 g (29 % d.Th.)
Siedepunkt: 250° C/0,1 mbar

## Ansprüche

1. Substituierte Pyridine der Formel I

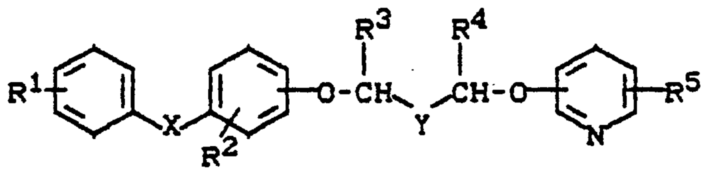

(I)

in welcher
X für O, S, NH, $-CH_2-$, $-CHCH_3-$, $-C(CH_3)_2-$steht,
Y für geradkettiges oder verzweigtes Alkylen, das gegebenenfalls durch Sauerstoff unterbrochen ist, steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam für eine direkte Bindung stehen können,
$R^5$ für Wasserstoff, Halogen, Alkyl, Halogenalkyl, Alkoxy, Amino, Alkyl, Alkylamino, Dialkylamino, Acylamino steht,
sowie deren Salze mit Säuren.

2. Verfahren zur Herstellung der substituierten Pyridine der Formel I

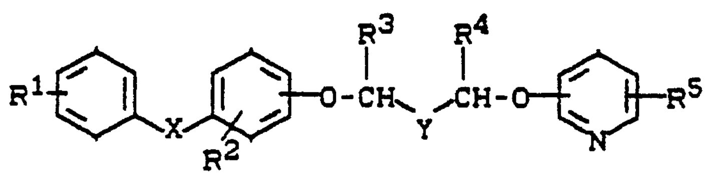

(I)

in welcher
X für O, S, NH, $-CH_2-$, $-CHCH_3-$, $-C(CH_3)_2-$steht,
Y für geradkettiges oder verzweigtes Alkylen, das gegebenenfalls durch Sauerstoff unterbrochen ist, steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam für eine direkte Bindung stehen können,
$R^5$ für Wasserstoff, Halogen, Alkyl, Halogenalkyl, Alkoxy, Amino, Alkyl, Alkylamino, Dialkylamino, Acylamino steht,
sowie deren Salze mit Säuren, die dadurch gekennzeichnet sind, daß man

a) Verbindungen der Formel II

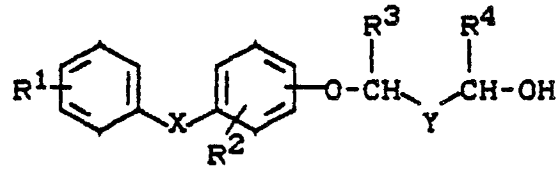

in welcher
$R^1$, $R^2$, $R^3$, $R^4$, X, Y die oben angegebene Bedeutung haben,
mit Halogenpyridinen der Formel III

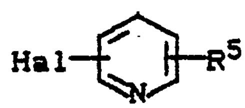

III

L1,5

in welcher
Hal für Halogen steht und
$R^5$ die oben angegebene Bedeutung hat,
umsetzt, oder

b) Verbindung der Formel IV

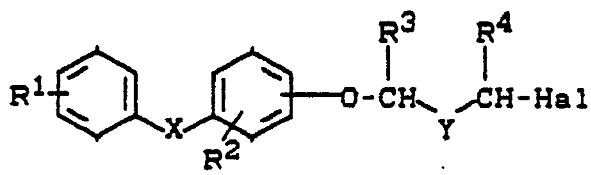

IV

in welcher
$R^1$, $R^2$, $R^3$, $R^4$, X, Y die oben angegebene Bedeutung haben, und
Hal für Halogen steht,
mit Hydroxypyridinen der Formel V

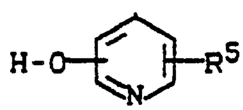

V

in welcher
$R^5$ die oben angegebene Bedeutung hat,
umsetzt.

3. Verbindungen der Formel II

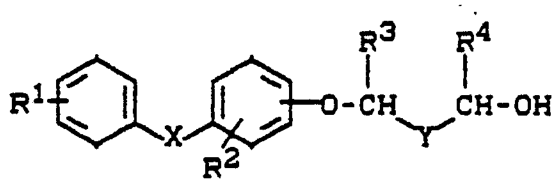

II

in welcher
$R^1$, $R^2$, $R^3$, $R^4$, X, Y die oben angegebene Bedeutung haben.

4. Verfahren zur Herstellung der Verbindungen der Formel II, dadurch gekennzeichnet, daß man Verbindungen der Formel VI

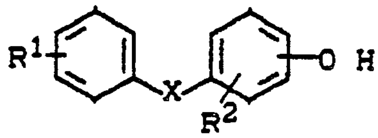

VI

in welcher
$R^1$, $R^2$, X die oben angegebene Bedeutung haben,
mit Verbindungen der Formeln VII oder VIII

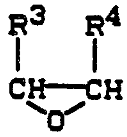

VII

in welcher
$R^3$ und $R^4$ die oben angegebene Bedeutung haben,

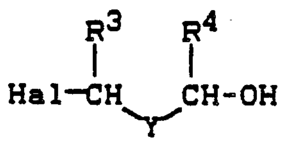

VIII

in welcher
Hal, $R^3$, $R^4$, Y die oben angegebene Bedeutung haben.
umsetzt.

5. Verwendung von substituierten Pyridinen der allgemeinen Formel I gemäß Anspruch 1 zur Bekämpfung von Insekten, insbesondere von Flöhen.

6. Mittel zur Bekämpfung von Insekten, insbesondere von Flöhen, gekennzeichnet durch einen Gehalt an mindestens einem substituierten Pyridin der Formel I, gemäß Anspruch 1.

7. Verfahren zur Bekämpfung von Insekten, insbesondere von Flöhen, dadurch gekennzeichnet, daß man substituierte Pyridine der Formel I gemäß Anspruch 1 auf Flöhe, ihre Wirtstiere und/oder ihren Lebensraum einwirken läßt.

8. Verfahren zur Herstellung von Mitteln zur Bekämpfung von Insekten, insbesondere von Flöhen, dadurch gekennzeichnet, daß man substituierte Pyridine der Formel I gemäß Anspruch 1 mit Streckmitteln und/oder oberflächenaktiven Mitteln vermischt.

9. Verwendung von substituierten Pyridinen der Formel I gemäß Anspruch 1 zur Herstellung von Bekämpfungsmitteln gegen Insekten, insbesondere Flöhe.